# EUROPEAN PATENT APPLICATION

(11) **EP 2 815 711 A1**
(43) Date of publication of application: **24.12.2014**
(21) Application number: 13748571.0
(22) Date of filing: 06.02.2013
(51) Int. Cl.: A61B 18/02, A61M 1/00, A61M 27/00

(54) **BRAIN COOLING DEVICE ALSO HAVING FLUID DISCHARGE FUNCTION**

(30) Priority: 15.02.2012 KR 20120015556
(71) Applicant: Kyungpook National University Industry-Academic Cooperation Foundation, Daegu 702-701 (KR)
(72) Inventor: PARK, Jae Chan, Daegu 706-777 (KR); SUK, Kyoung Ho, Daegu 706-952 (KR); HAN, Hyung Soo, Daegu 706-935 (KR); RHEE, Dong Ick, Seongnam-si Bundang-gu Gyeonggi-do 463-480 (KR)
(74) Representative: Hughes, Andrea Michelle
(86) International application number: PCT/KR2013/000957
(87) International publication number: WO 2013/122349

(57) **Abstract**

In a brain cooling device according to an embodiment of the present invention, preferably, temperature sensors are installed in one end and the other end of a cooling fluid transport tube to detect a temperature, a pressure measurement sensor is installed in a storage tank, a control unit is connected to the temperature sensors and the pressure measurement sensor, and the control unit calculates an amount of heat absorbed from a brain according to a change in pressure of the storage tank and the temperature detected by the temperature sensors. In the brain cooling device according to the embodiment of the present invention, preferably, one end of the cooling fluid transport tube and a drainage tube is bent such that adjacent drainage tubes come in close contact with each other. The brain cooling device according to the present invention is directly installed in a dura mater of a brain or a site in which an operation of incising the dura mater of the brain is performed so that heat or swelling is effectively mitigated. After use of the device ends, the brain cooling device may be removed through an operation hole which allows a cooling fluid to be introduced and discharged and penetrates a scalp and a cranium and thereby the device may be removed without a separate operation. When vaporized liquid nitrogen and the like are used as the cooling fluid, it is possible to cool the brain without separate mechanical and electric devices. In addition, blood and the like accumulated in an operation area may be discharged using a drain hole installed in the cooling device.

## Description

### CROSS-REFERENCE TO RELATED APPLICATION

This application claims priority to and the benefit of Korean Patent Application No. 10-2012-0015556, filed on February 15, 2012, the disclosure of which is incorporated herein by reference in its entirety.

### BACKGROUND

### 1. Field of the Invention

The present invention relates to a brain cooling device having a drainage function, and more particularly, to a device capable of cooling a brain on which an operation is performed in order to suppress edema and damage of the brain for a patient immediately after a craniotomy and having a drainage function for preventing blood and the like from accumulating in an operation site.

### 2. Discussion of Related Art

In various types of brain damage such as cerebral infarctions, cerebral hemorrhages, and head injuries, when hypothermia treatment is performed to decrease a temperature of the brain, a treatment effect of mitigating cerebral edema, an intracranial pressure increase, and the like due to diseases may be induced.

In general, in order to decrease a temperature of the brain, a temperature of the whole body is decreased, thereby decreasing a temperature of the brain. Directly decreasing a temperature of the head is difficult since the cranium, the scalp, and the like surrounding the brain interfere with effective temperature decrease in the brain.

However, when a craniotomy is performed to treat the above diseases, only a temperature of the brain may be locally decreased using an operation site rather than the whole body. Also, when blood and the like accumulate in the craniotomy site immediately after the operation and press on the brain, a reoperation may be necessary. Therefore, a drainage tube is generally installed in order to continuously discharge blood and the like from the operation site using negative pressure.

### SUMMARY OF THE INVENTION

According to the present invention, a cooling device is installed through an operation site when a craniotomy is performed, a temperature of a brain decreases during a necessary period after the operation, and when a treatment period ends, the cooling device inserted into the cranium may be easily removed in a bed without a reoperation.

In addition, a function of a drainage tube configured to continuously discharge blood and the like accumulated in the operation site is performed.

The object of the present invention is not limited to the above description, and other unmentioned objects may be clearly understood by those skilled in the art from the following description.

According to an aspect of the present invention, there is provided a brain cooling device for a craniotomy patient having a drainage function which is installed in a brain or a dura mater to cool the brain. The device includes a cooling fluid transport tube having a tube shape whose one end communicates with the other end and having a contact unit that is buried in contact with the brain or the dura mater; and a drainage tube connected to one end of the cooling fluid transport tube.

In the drainage tube, a plurality of drain holes may be formed in locations corresponding to the contact unit, and one end of the drainage tube may pass through a scalp and a cranium and may be exposed to the outside.

The contact unit may be bent and formed in the form of a zigzag.

A normal saline solution may be introduced into the cooling fluid transport tube.

Temperature sensors may be installed in one end and the other end of the cooling fluid transport tube to detect a temperature of the normal saline solution introduced into the cooling fluid transport tube and a temperature of a discharged normal saline solution.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 is a plan view and a cross sectional view of a brain cooling device for a craniotomy patient having a drainage function according to an embodiment of the present invention;
FIG. 2 is a conceptual diagram illustrating an installation state of the brain cooling device for a craniotomy patient having a drainage function illustrated in FIG. 1; and
FIGS. 3 and 4 are conceptual diagrams illustrating a state in which the brain cooling device for a craniotomy patient having a drainage function illustrated in FIG. 1 is installed and then suturing is performed.

### DETAILED DESCRIPTION OF EXEMPLARY EMBODIMENTS

Hereinafter, a brain cooling device for a craniotomy patient having a drainage function (hereinafter referred to as a "brain cooling device") according to the present invention will be described in detail with reference to the accompanying drawings.

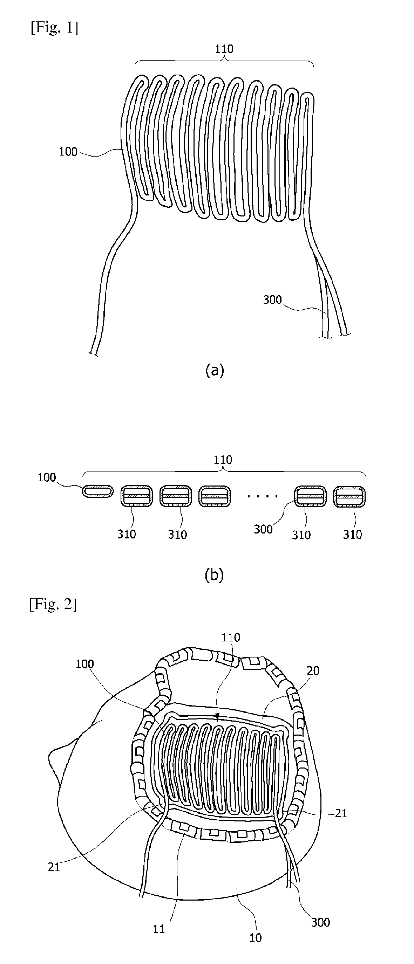

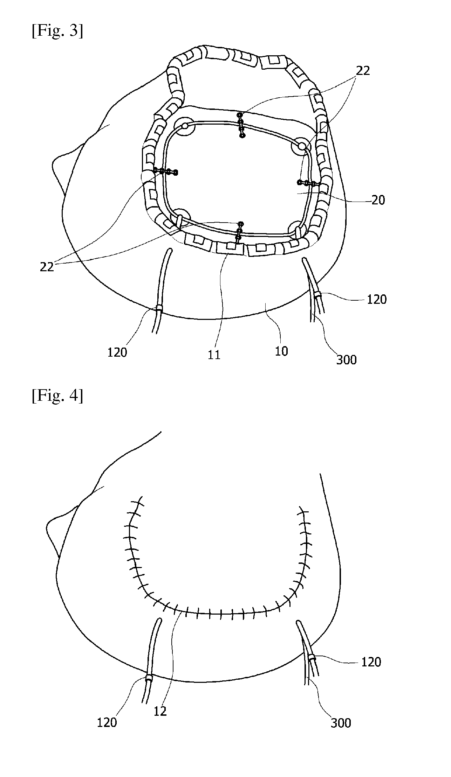

FIG. 1 is a plan view and a cross sectional view of a brain cooling device for a craniotomy patient having a drainage function according to an embodiment of the present invention. FIG. 2 is a conceptual diagram illustrating an installation state of the brain cooling device for a craniotomy patient having a drainage function illustrated in FIG. 1. FIGS. 3 and 4 are conceptual diagrams illustrating a state in which the brain cooling device for a craniotomy patient having a drainage function illustrated in FIG. 1 is installed and then suturing is performed.

As illustrated in FIG. 1, the brain cooling device according to the present invention is a cooling device installed in a brain or a dura mater and configured to cool the brain. The brain cooling device includes a cooling fluid transport tube 100 having a tube shape whose one end communicates with the other end and having a contact unit 110 that is buried in contact with the brain or the dura mater, and a drainage tube 300 disposed in one end of the cooling fluid transport tube 100 and having a plurality of drain holes 310.

Cooling water such as a normal saline solution flows in the fluid transport tube 100. One end and the other end thereof are open, and the cooling water is introduced into the one end and discharged from the other end.

As illustrated in FIG. 1, the fluid transport tube 100 has the contact unit 110 that is bent in the form of a zigzag. As illustrated in FIG. 2, the contact unit 110 is bent and formed in the form of a zigzag and is disposed in contact with the brain or the dura mater while a scalp 10 and a cranium 20 of a patient are incised and opened.

As illustrated in FIG. 1, the drainage tube 300 is connected below the fluid transport tube 100. The plurality of drain holes 310 are formed in the drainage tube 300 of a part corresponding to the contact unit 110 in contact with the brain or the dura mater, as illustrated in FIG. 1(b).

As illustrated in FIG. 1(b), preferably, the drainage tube 300 is formed to have a closed other end and is able to apply a negative pressure to an inside.

As illustrated in FIG. 3, the cooling fluid transport tube 100 described above preferably has a rectangular cross section in close contact with the brain or the dura mater. The drainage tube 300 connected thereto may be formed in various shapes, but preferably has a rectangular cross section as in the cooling fluid transport tube 100 to effectively drain blood, body fluid, and the like.

FIGS. 3 and 4 illustrate a state in which the brain cooling device according to the embodiment of the present invention is installed and then the cranium is sutured. As illustrated, when the opened cranium 20 is closed using an engaging member 22 that is separately provided, the cooling fluid transport tube to which the drainage tube 300 is connected is exposed to the outside of the cranium through a perforated portion 21 of the cranium 20.

In this case, preferably, temperature sensors 120 are installed in one end and the other end of the cooling fluid transport tube 100 such that a temperature of a normal saline solution introduced into the cooling fluid transport tube 100 and a temperature of a normal saline solution discharged therefrom are detected to monitor a cooling state of the brain or the dura mater.

The above-described cooling fluid transport tube 100 and drainage tube 300 are preferably made of a soft material to be easily removed in a bed. That is, in a state illustrated in FIG. 4, preferably, when one end of the cooling fluid transport tube 100 and the drainage tube 300 is pulled, the bent fluid transport tube 100 and the drainage tube 300 gently spread and are discharged through the perforated portion 21 of the cranium 20.

As described above, in a brain cooling device for a craniotomy patient according to the present invention, a contact unit in direct contact with a brain or a dura mater is installed and cooling of the brain or the dura mater may be effectively performed using a cooling material flowing into the contact unit. Also, the cooling unit may be easily removed in the bed. In addition, without using a separate drainage tube, the drainage tube is connected to the cooling fluid transport tube and thereby blood, a body fluid, and the like accumulated in an operation site may be effectively discharged.

Effects of the present invention are not limited to the above description, and other unmentioned effects may be clearly understood by those skilled in the art from the following description.

When a craniotomy is performed to treat various types of brain damage such as cerebral infarctions, cerebral hemorrhages, and head injuries, if the present invention is applied to hypothermia treatment for mitigating cerebral edema, an intracranial pressure increase, and the like, the brain or the dura mater may be effectively cooled and a cooling unit may be easily removed in the bed. In addition, without using a separate drainage tube, the drainage tube is connected to the cooling fluid transport tube and thereby blood, a body fluid, and the like accumulated in an operation site may be effectively discharged. When a craniotomy is performed to treat brain damage, the present invention may be used in the field of medical devices that locally decrease a temperature of only the brain rather than the whole body and increase efficiency of treatment.

Embodiments described in this specification and the accompanying drawings are only examples describing a part of the scope and spirit of the present invention. Therefore, the embodiments disclosed herein are provided to describe the scope and spirit of the present invention and not for purposes of limitation. Therefore, it is apparent that the present invention is not limited to these embodiments. It should be understood that modifications and detailed embodiments that can be easily inferred by those skilled in the art within the scope and spirit included in the specification and drawings of the present invention fall within the scope of the present invention.

## Claims

1. A brain cooling device for a craniotomy patient having a drainage function which is installed in a brain or a dura mater to cool the brain, the device comprising:
a cooling fluid transport tube having a tube shape whose one end communicates with the other end and having a contact unit that is buried in contact with the brain or the dura mater; and
a drainage tube connected to one end of the cooling fluid transport tube.

2. The brain cooling device of claim 1,
wherein, in the drainage tube , a plurality of drain holes are formed in locations corresponding to the contact unit, and one end of the drainage tube passes through a scalp and a cranium and is exposed to the outside.

3. The brain cooling device of claim 1,
wherein the contact unit is bent and formed in the form of a zigzag.

4. The brain cooling device of claim 1,
wherein a normal saline solution is introduced into the cooling fluid transport tube.

5. The brain cooling device of claim 4,
wherein temperature sensors are installed in one end and the other end of the cooling fluid transport tube to detect a temperature of the normal saline solution introduced into the cooling fluid transport tube and a temperature of a discharged normal saline solution.
